# EUROPÄISCHE PATENTSCHRIFT

(11) **EP 1 868 966 B1**
(45) Veröffentlichungstag und Bekanntmachung des Hinweises auf die Patenterteilung: **17.06.2020**
(21) Anmeldenummer: 06707323.9
(22) Anmeldetag: 28.02.2006
(51) Int. Cl.: C07C 1/20, C07C 11/02, C07C 11/06

(54) **VERFAHREN ZUR HERSTELLUNG VON C2- BIS C4-OLEFINEN AUS EINEM OXYGENATE UND WASSERDAMPF ENTHALTENDEN EINSATZSTROM**
PROCESS FOR PREPARING C2 TO C4 OLEFINS FROM A FEED STREAM COMPRISING OXYGENATES AND STEAM
PROCEDE POUR PRODUIRE DES OLEFINES C2-C4 A PARTIR D'UN FLUX D'ENTREE COMPRENANT DES COMPOSES OXYGENES ET DE LA VAPEUR D'EAU

(30) Priorität: 06.04.2005 DE 102005015923
(43) Veröffentlichungstag der Anmeldung: 26.12.2007
(73) Patentinhaber: Air Liquide Global E&C Solutions Germany GmbH, 60439 Frankfurt am Main (DE)
(72) Erfinder: ROTHAEMEL, Martin, 60437 Frankfurt/Main (DE); BOLL, Walter, 60388 Frankfurt/Main (DE); BIRKE, Gerhard, 60389 Frankfurt/Main (DE); KOEMPEL, Harald, 63263 Neu-Isenburg (DE); LIEBNER, Waldemar, 61440 Oberursel (DE); BACH, Hermann, 56412 Heiligenroth (DE)
(86) Internationale Anmeldenummer: PCT/EP2006/001817
(87) Internationale Veröffentlichungsnummer: WO 2006/105831

(56) Entgegenhaltungen:
- WO-A-97/36845
- DE-A1- 3 524 890
- DE-A1- 10 233 975

## Beschreibung

Die Erfindung betrifft ein Verfahren zur Herstellung von C₂- bis C₄-Olefinen, vorzugsweise Propylen, aus einem dampfförmige Oxygenate, vorzugsweise Methanol- und/oder Dimethylether, und Wasserdampf enthaltenden, eine Temperatur von 280 bis 480 °C besitzenden Einsatzstrom, der über mindestens eine in einem Reaktor angeordnete aus einer Schüttung aus körnigem, formselektiven Zeolith-Katalysator vom Pentasiltyp gebildete Festbettzone geleitet und die Oxygenate bei einer Reaktionstemperatur von 350 bis 550 °C, vorzugsweise 420 bis 490 °C, zu Olefinen mit hoher Selektivität für niedrige Olefine katalytisch umgesetzt werden und das aus dem Reaktor austretende Reaktionsgemisch in einen C₂- bis C₃- Olefine, vorzugsweise Propylen, enthaltenden ersten Produktstrom, in mindestens einen weiteren C₄₊-Olefine enthaltenden zweiten Produktstrom und in einen aus wässrige Phase bestehenden dritten Produktstrom getrennt wird.

Aus der DE-A-19723363 ist ein Verfahren zur Herstellung von C₂- und C₃-Olefinen durch Umsetzen eines Methanol- und/oder Dimethyletherdampf und Wasserdampf enthaltenden Reaktionsgemisches in einem indirekt gekühlten Röhrenreaktor an einem formselektiven Festbett-Katalysator bei Temperaturen von 280 bis 570 °C und Drücken von 0,1 bis 1 bar bekannt. Aus dem Reaktor wird ein C₂- bis C₄-Olefine und C₅₊-Olefine enthaltender Produktstrom abgezogen, gekühlt und in ein C₂- und C₃-Olefine und ein C₅₊-Olefine aufweisendes Produkt getrennt.

Eine Weiterentwicklung dieses Verfahrens besteht gemäß DE-A-10027159 darin, dass Methanol zunächst an einem in einem Reaktorbehälter befindlichen Festbett-Katalysator aus körnigem Al₂O₃ zu einem Dimethylether enthaltenden Dampfgemisch umgesetzt wird. Zur Herstellung eines C₃-Olefine enthaltenden Produkts wird ein erster Teilstrom des Dampfgemisches zusammen mit Wasserdampf in einen mit einem Festbett aus formselektivem Zeolith-Katalysator gefüllten ersten Reaktionsbehälter geleitet und das aus diesem abgeführte Zwischenprodukt zusammen mit einem zweiten Teilstrom des vorstehend angeführten Dampfgemisches einem mit einem Festbett aus formselektiven Zeolith-Katalysator gefüllten zweiten Reaktionsbehälter aufgegeben. Das aus dem zweiten Reaktionsbehälter abgezogene Produkt wird in einen ca. 97 Vol.-% C₃-Olefine und einen Reststoffe, wie C₂-Olefine und C₄₊-Olefine, enthaltenden Produktstrom getrennt, wobei der Reststoff-Produktstrom in mindestens einen der mit Zeolith-Katalysator gefüllten Reaktionsbehälter zurückgeführt wird.

Bei einem in der DE-A-19648795 beschriebenen Verfahren zur Herstellung von C₃- und C₄-Olefinen wird ein C₄- bis C₇-Olefine enthaltender Einsatzstrom an einem in einen Reaktionsbehälter eingebrachten formselektiven Zeolith-Katalysator vom Pentasiltyp in ein C₃- und C₄-Olefine enthaltendes Produkt, vorzugsweise adiabatisch, umgesetzt. Anschließend wird dieses Produkt gekühlt, so dass Wasser und Benzin auskondensieren. Das gebildete Kondensat wird in eine wasserhaltige Phase, eine organische Flüssigphase und eine C₂- bis C₄-Olefine sowie in geringe Anteile Paraffine aufweisende gasförmige Phase getrennt, die gasförmige Phase zur Abtrennung von C₂- und C₃-Olefinen durch eine Trenneinrichtung geleitet und die organische Flüssigphase mittels Destillation in eine C₃-, C₄-Olefine und kleinen Mengen an gesättigten Kohlenwasserstoffen und in eine Benzin enthaltende Phase zerlegt. Von der C₃-, C₄-Olefine und kleine Mengen an gesättigten Kohlenwasserstoffen aufweisenden Phase können neben Propylen vor allem Ethylen, n-Buten-1 und Isobutylen destillativ oder adsorptiv abgetrennt werden.

Die US-A-6441261 bezieht sich auf ein Verfahren zum Umsetzen eines ein Oxygenat und ein inertes Verdünnungsmittel enthaltenden Einsatzstroms zu einem Olefin-Produkt, indem der Einsatzstrom über einen in einem Reaktorbehälter angeordneten Silizium-Aluminophosphat-Katalysator, vor dem der Druck des Einsatzstroms 12 bis 42 at und der Partialdruck des Oxygenats von 1 bis 5 at betragen, geleitet wird.

Die DE-Offenlegungsschrift DE 35 24 890 A1 offenbart ein Verfahren zur Herstellung niedermolekularer Olefine, bei dem man Methanol und/oder Dimethylether zusammen mit rückgeführten Kohlenwasserstoffen in der Gasphase in Kontakt mit einem Zeolith-Katalysator bringt und so Methanol und/oder Dimethylether in niedermolekulare Olefine umwandelt, und die Gesamtheit oder einen Teil des nach Entfernung von gebildetem Ethylen und Propylen aus dem Reaktionsprodukt verbleibende Reaktionsprodukt als die rückgeführten Kohlenwasserstoffe verwendet.

Die DE-Offenlegungsschrift DE 102 33 975 A1 beschreibt eine Vorrichtung zur Herstellung von Propylen aus Methanol, bestehend aus einer mit einem hochaktiven und hochselektiven gamma-Aluminiumoxid-Katalysator ausgestatteten Vor-Reaktionsstufe zur Bildung eines Methanol-, Dimethylether (DME)- und Wasserdampf enthaltenden Vor-Reaktions-Gemisches aus Methanol bei Betriebstemperaturen von 250 bis 460 °C, mehreren, vorzugsweise zwei oder drei hintereinander angeordneten jeweils mit einem formselektiven Zeolithkatalysator vom Pentasil-Typ ausgestatteten Reaktionsstufen zur Umsetzung des Vor-Reaktions-Gemisches bei Eintrittstemperaturen von 400 bis 460 °C und Drücken von 0,5 bis 3,0 bar(abs), Wärmetauschern zwischen den Reaktionsstufen zur Abkühlung des austretenden Reaktionsgemisches auf eine Temperatur von 400 bis < 460 °C, mehrere nach der letzten Reaktionsstufe angebrachte Wäermetauscher zur Abkühlung des austretenden Reaktions-Gemisches auf eine Temperatur von 100 bis 200 °C, einer anschliessenden Trennvorrichtung zum Trennen des Reaktionsgemisches in eine Gas- und eine überwiegend Wasser enthaltende Flüssigkeitsphase, einem Kompressor zum Verdichten der Gasphase, einer Trennvorrichtung zum Trennen des bei der Verdichtung gebildeten Gemisches in eine Kohlenwasserstoffe enthaltende Gasphase, eine DME, Wasser und Methanol enthaltende Flüssigkeitsphase und eine Kohlenwasserstoffe enthaltende Flüssigkeitsphase und einer Trennvorrichtung zum Trennen der Kohlenwasserstoffe enthaltenden Phase in Propylen und andere Kohlenwasserstoffe, wobei die mit dem Zeolithkatalysator ausgestatteten Reaktionsstufen senkrecht übereinander in einem stehenden Reaktorbehälter angeordnet sind und von oben nach unten durchströmbar sind, wobei jeweils zwischen den Reaktionsstufen Wärmetauscher angebracht sind, deren Eintrittsöffnungsquerschnitt dem Querschnitt der Austrittsöffnung der Reaktionsstufe entspricht und die unmittelbar an die Unterseite der Reaktorstufe anschliessen oder in geringem Abstand von dieser angeordnet sind.

Es ist die Aufgabe der vorliegenden Erfindung, das eingangs beschriebene Verfahren so zu verbessern, dass bei vermindertem Aufwand eine möglichst große Ausbeute an C₂- bis C₄-Olefinen, ganz besonders jedoch an Propylen, durch katalytische Umsetzung von Oxygenaten erzielt wird.

Gelöst ist diese Aufgabe im Wesentlichen durch ein Verfahren mit den Merkmalen des Anspruchs 1. Durch die dort offenbarte Regelung wird fortlaufend die Temperatur des Einsatzstroms am Eintritt in die Festbettzone erfasst und mit dem Temperatur-Sollwert für das aus der Festbettzone austretende Reaktionsgemisch verglichen und so die Temperatur des Einsatzstroms im Hinblick auf die Temperatur der katalytischen Umsetzung durch in den Einsatzstrom eingeleiteten Zusatzstrom beeinflusst.

Aufgrund der hohen Wärmekapazität der Olefine und inerten Gaskomponenten des Zusatzstroms sowie möglicher endothermer Reaktionen durch die katalytische Spaltung höherer Kohlenwasserstoffe, z.B. C₆H₁₂ → 2 C₃H₆, sinkt die adiabate Temperaturerhöhung im Festbett, so dass bei vorgegebenem Temperatur-Sollwert für das aus dem Festbett abgeführte Reaktionsgemisch die Temperatur am Eintritt des Einsatzstroms in das Festbett angehoben werden kann. Dabei werden sowohl die Selektivität der C₂- bis C₄-Olefine aus der Umsetzung der Oxygenate als auch aufgrund der Temperaturabhängigkeit des Umsetzungsgrads selbst der Umsatz der Oxygenate gesteigert. Ingesamt wird dadurch eine Erhöhung der Ausbeute an C₂- bis C₄-Olefinen erzielt.

Zur Erhöhung der Ausbeute an C₂- bis C₄-Olefinen ist es weiter von Vorteil, wenn die C₄₊-Olefine des zweiten Produktstroms und die inerten Gaskomponenten des ersten Produktstroms zusammengeführt als Zusatzstrom in den Einsatzstrom rückgeführt werden.

Bei den inerten Gaskomponenten handelt es sich um einen oder mehrere der Stoffe Wasserdampf, Stickstoff, Helium, Neon, Argon, Wasserstoff, Kohlenmonoxid, Kohlendioxid und C₁- bis C₄-Paraffine, durch die der Partialdruck der Reaktionskomponenten abgesenkt und dadurch die Selektivität und Ausbeute der C₂- bis C₄-Olefine, insbesondere des Propylens, gesteigert werden.

Die Ausbeute an C₂- bis C₄-Olefinen ist optimierbar, wenn Zusatzströme eingesetzt werden, die eine oder mehrere der Komponenten C₂- bis C₈-Olefine, vorzugsweise C₂-Olefine und C₄- bis C₆-Olefine, C₁- bis C₈-Paraffine, vorzugsweise C₁- bis C₆-Paraffine, C₅- bis C₈-Naphthene, vorzugsweise C₅- bis C₆-Naphthene sowie C₆- bis C₇-Aromaten enthalten.

Auch die beim Betrieb von thermischen und katalytischen Crackern oder dergl. Prozessen anfallenden Nebenproduktströme können als Zusatzströme verwendet werden.

Ebenso lassen sich prozessinterne Recyclingströme als Zusatzströme einsetzen.

Die Durchsatzmenge der in dem Einsatzstrom enthaltenen Oxygenate beträgt 0.1 bis 10 kg, vorzugsweise 0.3 bis 1.5 kg pro Stunde und pro kg eingesetztem Katalysator.

Eine vorzugsweise Ausgestaltung des erfindungsgemäßen Verfahrens besteht darin, dass der Einsatzstrom einem Reaktor mit mehreren, vorzugsweise mit zwei bis sechs Festbettzonen aufgegeben wird, indem der Einsatzstrom in eine der Zahl der Festbettzonen entsprechende Zahl von Einsatzteilströmen aufgeteilt und jeder Einsatzteilstrom einer korrespondierenden Festbettzone zugeleitet wird, wobei das aus einer Festbettzone ausgetragene Reaktionsgemisch der nächst folgenden Festbettzone und das aus der stromabwärts liegenden letzten Festbettzone ausgetragene Reaktionsgemisch einer Trennanlage zugeführt wird. Ein Olefine und inerte Gaskomponenten enthaltender Zusatzstrom wird in eine der Zahl der Einsatzteilströme entsprechende Zahl von Zusatzteilströmen aufgeteilt und jeder Zusatzteilstrom in einen korrespondierenden Einsatzteilstrom bzw. in das aus einer Festbettzone austretende und der nächstfolgenden Festbettzone aufgegebene Reaktionsgemisch eingeleitet.

Die Erfindung wird durch ein Ausführungsbeispiele und an Hand der beigefügten Zeichnungen näher erläutert.

Es zeigen:
- Fig. 1: ein Verfahrensfließbild mit einem Reaktor und dem Regelschema
- Fig. 2: bei Betrieb des Verfahrens mit einem Reaktor gemessenen Temperaturprofile
- Fig. 3: ein Verfahrensfließbild mit drei in einem Reaktor angeordneten Festbettzonen und dem Regelschema

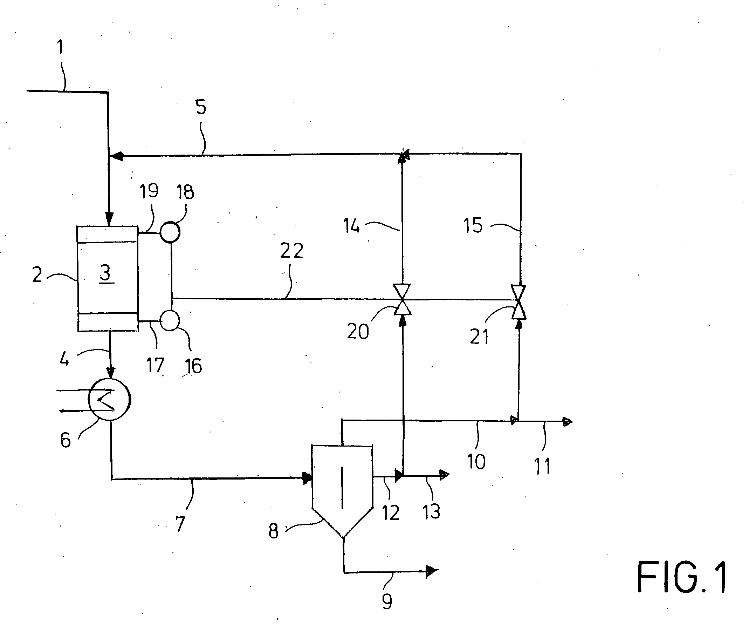

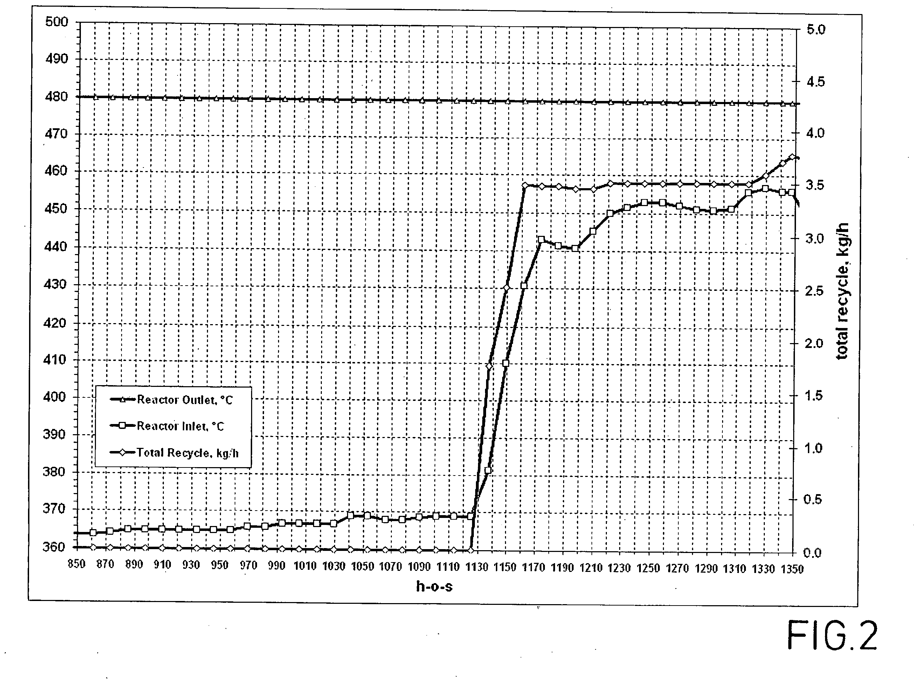

Gemäß Fig. 1 und Fig. 2 wird ein dampfförmiger, Oxygenate und Wasserdampf enthaltender, eine Temperatur von 364 - 369 °C aufweisender Einsatzstrom über Leitung (1) dem Reaktor (2) aufgegeben (Fig. 2, h-o-s, reactor inlet, °C, bis zu einer Betriebszeit von 1125 h-o-s [hours-on-stream]). In dem Reaktor (2) ist eine eine Festbettzone (3) bildende Schüttung aus körnigem Zeolith-Katalysator vom Pentasiltyp mit formselektiven Eigenschaften und mit einer Korngröße von 1 bis 8 mm angeordnet ist. Beim Durchleiten des Einsatzstroms durch die Festbettzone (3) erfolgt eine adiabatische Umsetzung, so dass die Temperatur des über Leitung (4) den Reaktor (2) verlassenden Reaktionsgemisches mit 480 °C (Fig.2, h-o-s, reactor outlet, °C, bis zu einer Betriebszeit von 1125 h-o-s) konstant bleibt. Nachdem über Leitung (5) ein olefinhaltiger und inerte Gaskomponenten aufweisender, prozessintern gebildeter Zusatzstrom in einer Menge von 3.5 kg/h in den Einsatzstrom (Fig.2, total recycle kg/h) rückgeführt wird, kann die Temperatur des den Reaktor verlassenden Reaktionsgemisches auf den Sollwert von 480 °C eingeregelt werden, indem die Eintrittstemperatur auf 420 bis 430 °C angehoben wird. Das über Leitung (4) aus dem Reaktor (2) austretende Reaktionsgemisch wird in einem Kühler (6) auf eine Temperatur im Bereich von 25 bis 80 °C gekühlt, so dass Wasser und Kohlenwasserstoffe, insbesondere Benzin, auskondensieren. Das Kondensat wird über Leitung (7) einem Abscheider (8) zugeführt, in dem eine Trennung in einen C₂- bis C₃-Olefine enthaltenden gasförmigen ersten Produktstrom, in einen C₄₊-Olefine enthaltenden zweiten Produktstom und in einen wässrige Oxygenate enthaltenden dritten Produktstrom erfolgt. Über Leitung (9) wird der die wässrigen Oxygenate enthaltende dritte Produktstrom aus dem Prozess ausgeleitet. Aus dem über Leitung (10) aus dem Abscheider (8) abgezogenen ersten Produktstrom wird Propylen destillativ abgetrennt und als Hauptprodukt über Leitung (11) aus dem Prozess ausgeleitet. Der über Leitung (12) abgeführte zweite Produktstrom wird destillativ in C₆-Olefine und in C₇₊-Olefine getrennt, wobei die C₇₊-Olefine über Leitung (13) aus dem Prozess ausgeleitet, die C₆-Olefine über Leitung (14) in Leitung (5) und der aus C₂-Olefinen und inerten gasförmigen Komponenten gebildete Reststrom über Leitung (15) in Leitung (5) eingespeist werden. Die in Leitung (5) zusammengeführten Stoffströme bilden den Zusatzstrom, der dem Oxygenate enthaltenden über Leitung (1) dem Reaktor (2) zugeführten Einsatzstrom zugegeben wird.

Der Sollwert der Temperatur von 480 °C für das aus dem Reaktor (2) austretende Reaktionsgemisch wird mittels eines unterhalb des Katalysator-Festbetts (3) angebrachten mit einem Messwertgeber (16) verbundenen Temperaturfühlers (17) und die Temperatur des in den Reaktor (2) eintretenden das Katalysator-Festbett (3) durchströmenden Einsatzstroms mittels eines oberhalb des Katalysator-Festbetts (3) angeordneten mit einem Messwertgeber (18) verbundenen Temperaturfühlers (19) gemessen. Der Messwert der Eintrittstemperatur des Einsatzstroms wird mit dem vorgegebenen Sollwert der Austrittstemperatur des Reaktionsgemisches verglichen; falls die Eintrittstemperatur des Einsatzstroms zu klein ist, werden die in den Leitungen (14,15) angeordneten Durchflussregler (20,21) über Leitung (22) elektrisch angesteuert, geöffnet und die Temperatur des Einsatzstroms beeinflussender olefinehaltiger Zusatzstrom dem Einsatzstrom zugesetzt. Nach dem Einleiten der erforderlichen Menge an Zusatzstrom werden die Durchflussregler (20,21) wieder geschlossen.

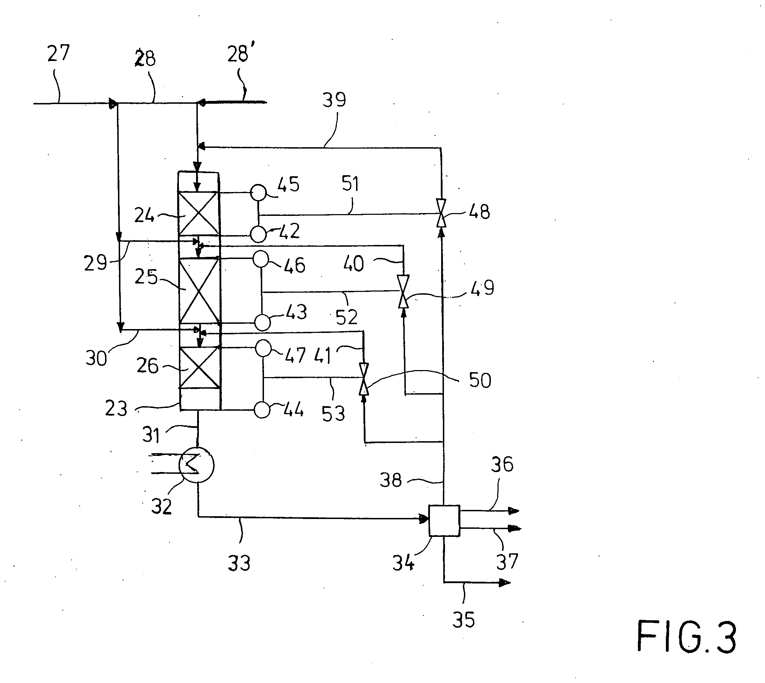

Bei dem in Fig. 3 dargestellten Verfahrensfließbild befinden sich in einem Reaktor (23) drei übereinander angeordnete Festbettzonen (24,25,26), bestehend jeweils aus einer Schüttung von körnigem Zeolith-Katalysator vom Pentasiltyp mit formselektiven Eigenschaften und mit einer Korngröße von 1 bis 8 mm. Der über Leitung (27) ankommende, dampfförmige Oxygenate und Wasserdampf enthaltende Einsatzstrom wird mit einem über Leitung (28') zugeführten, aus inerten Komponenten gebildeten Gasstrom gemischt und dann auf eine Temperatur von 364 °C aufgeheizt. Anschließend wird der so gemischte Einsatzstrom in drei Einsatzteilströme aufgeteilt, von denen ein Einsatzteilstrom über Leitung (28) der oberen Festbettzone (24), ein zweiter Einsatzteilstrom über Leitung (29) der mittleren Festbettzone (25) und ein dritter Einsatzteilstrom über Leitung (30) der unteren Festbettzone (26) zugeführt wird. Das aus der oberen Festbettzone (24) austretende Reaktionsgemisch wird der mittleren Festbettzone (25) und das aus der mittleren Festbettzone (25) austretende Reaktionsgemisch der unteren Festbettzone (26) zugeführt. Das über Leitung (31) aus der unteren Festbettzone (26) abgeleitete Reaktionsgemisch wird in einem Kühler (32) auf eine Temperatur von 25 bis 80 °C gekühlt, so dass Wasser und Kohlenwasserstoffe, insbesondere Benzin, kondensieren. Über Leitung (33) gelangt das Kondensat in einer Trennanlage (34), in der dieses in einen gasförmigen, C₂- und C₃-Olefine enthaltenden ersten Produktstrom, einen C₄₊-Olefine aufweisenden zweiten Produktstrom und einen wässrige Oxygenate enthaltenden dritten Produktstrom getrennt wird. Der dritte oxygenatehaltige Produktstrom wird über Leitung (35) aus dem Prozess ausgleitet, von dem ersten Produktstrom wird Propylen als Hauptprodukt durch Destillation abgetrennt und dann über Leitung (36) aus dem Prozess ausgeleitet, der zweite Produktstrom wird durch Destillation in Ce-Olefine und C₇₊-Olefine zerlegt und die C₇₊-Olefine über Leitung (37) aus dem Prozess ausgeleitet. Die C₆-Olefine und die bei der Destillation des ersten Produktstroms gebildeten C₂-Olefine verlassen zusammengeführt über Leitung (38) die Trennanlage (34) und bilden den in den Prozess rückgeführten in drei Zusatzteilströme aufgeteilten Zusatzstrom. Die Zusatzteilströme werden jeweils anteilig über Leitung (39) dem über Leitung (28) der oberen Festbettzone (24) aufgegebenen Einsatzteilstrom, über Leitung (40) dem aus der oberen Festbettzone (24) austretenden Reaktionsgemisch und über Leitung (41) dem aus der mittleren Festbettzone (25) austretenden Reaktionsgemisch zugeleitet. Die Sollwerte der Temperatur für die aus den Festbettzonen (24,25,26) austretenden Reaktionsgemische werden jeweils mittels eines unterhalb des Festbetts angebrachten mit einem Messwertgeber (42,43,44) verbundenen Temperaturfühler und die Temperatur des der oberen Festbettzone (24) zugeführten Einsatzteilstroms sowie diejenige des der mittleren und unteren Festbettzone (25,26) aufgegebenen Reaktionsgemisches mit einem oberhalb der Festbettzone angeordneten mit einem Messwertgeber (45,46,47) verbundenen Temperaturfühler gemessen. Die in den Leitungen (39,40,41) für die Zusatzteilströme angeordneten Durchflussregler (48,49,50) werden über die Leitungen (51,52,53) elektrisch angesteuert.

## Patentansprüche

1. Verfahren zur Herstellung von C₂- bis C₄-Olefinen, vorzugsweise Propylen, aus einem dampfförmige Oxygenate, vorzugsweise Methanol und/oder Dimethylether, und Wasserdampf enthaltenden, eine Temperatur von 280 bis 480 °C besitzenden Einsatzstrom, der über mindestens eine in einem Reaktor (2) angeordnete, aus einer Schüttung aus körnigem, formselektiven Zeolith-Katalysator vom Pentasiltyp gebildete Festbettzone (3) geleitet und die Oxygenate bei einer Reaktionstemperatur von 350 bis 550 °C, vorzugsweise 420 bis 490 °C, zu Olefinen mit hoher Selektivität für niedrige Olefine katalytisch umgesetzt werden und das aus dem Reaktor austretende Reaktionsgemisch in einem Kühler (6) gekühlt, so dass Wasser und Kohlenwasserstoffe, insbesondere Benzin, auskondensieren, und in einem Abscheider (8) in einen C₂- bis C₃-Olefine enthaltenden ersten Produktstrom, in einen C₄₊-Olefine enthaltenden zweiten Produktstrom und in einen wässrige Oxygenate enthaltenden dritten Produktstrom getrennt wird, wobei Propylen aus dem ersten Produktstrom destillativ abgetrennt und als Hauptprodukt aus dem Verfahren ausgeleitet wird und wobei der wässrige Oxygenate enthaltende dritte Produktstrom aus dem Verfahren ausgeleitet wird, **dadurch gekennzeichnet, dass** am Eintritt des Einsatzstroms in die Festbettzone (3) ein Reaktionsdruck von 1.0 bis 3.0 bara und am Austritt des Reaktionsgemisches aus der Festbettzone ein Reaktionsdruck von 0.5 bis 2.0 bara herrschen und die Temperatur der katalytischen Umsetzung nach einem für das aus der Festbettzone austretenden Reaktionsgemisch vorgegebenen, im Bereich von 440 bis 520 °C liegenden Temperatur-Sollwert durch einen in den Einsatzstrom eingeleiteten, aus Olefinen und inerten Gaskomponenten bestehenden Zusatzstrom geregelt wird, wobei der zweite, C₄₊-Olefine umfassende Produktstrom destillativ in einen C₆-Olefine enthaltenden und in einen C₇₊-Olefine enthaltenden Strom getrennt wird, wobei die C₇₊-Olefine aus dem Verfahren ausgeleitet werden und die C₆-Olefine des zweiten Produktstroms mit den inerten Gaskomponenten und dem Ethylen des ersten Produktstroms zusammengeführt und als Zusatzstrom in den Einsatzstrom zurückgeführt werden, wobei die Temperatur des Einsatzstroms am Eintritt in die Festbettzone erfasst und mit dem Temperatur-Sollwert für das aus der Festbettzone austretende Reaktionsgemisch verglichen und so die Temperatur des Einsatzstroms im Hinblick auf die Temperatur der katalytischen Umsetzung durch in den Einsatzstrom eingeleiteten Zusatzstrom beinflusst wird.

2. Verfahren nach Anspruch 1, **dadurch gekennzeichnet, dass** der Temperatur-Sollwert im Bereich von 480 bis 495 °C liegt.

3. Verfahren nach einem der Ansprüche 1 bis 2, **dadurch gekennzeichnet, dass** als inerten Gaskomponenten einer oder mehrere der Stoffe Wasserdampf, Stickstoff, Helium, Neon, Argon, Wasserstoff, Kohlenmonoxid, Kohlendioxid und C₁- bis C₄-Paraffine eingesetzt werden.

4. Verfahren nach einem der Ansprüche 1 bis 3, **dadurch gekennzeichnet, dass** der Zusatzstrom eine oder mehrerer der Komponenten C₂- bis C₈- Olefine, vorzugsweise C₂-Olefine und C₄- bis C₆-Olefine, C₁- bis Ce-Paraffine, vorzugsweise C₁- bis C₆-Paraffine, C₅- bis C₈- Naphthene, vorzugsweise C₅- bis C₆-Naphthene, C₆- bis C₇-Aromaten enthält.

5. Verfahren nach einem der Ansprüche 1 bis 4, **dadurch gekennzeichnet, dass** die Durchsatzmenge der in dem Einsatzstrom enthaltenen Oxygenate 0.1 bis 10 kg, vorzugsweise 0.3 bis 1.5 kg pro Stunde und pro kg eingesetztem Katalysator beträgt.

6. Verfahren nach einem der Ansprüche 1 bis 5, **dadurch gekennzeichnet, dass** der einem Reaktor (23) mit mehreren, vorzugsweise zwei bis sechs Festbettzonen (24,25,26) zugeleitete Einsatzstrom in eine der Zahl der Festbettzonen entsprechende Zahl von Einsatzteilströmen aufgeteilt und jeder Einsatzteilstrom einer korrespondierenden Festbettzone aufgegeben wird, das aus einer Festbettzone ausgetragene Reaktionsgemisch der nächstfolgenden Festbettzone zugeführt und das aus der stromabwärts liegenden letzten Festbettzone ausgetragene Reaktionsgemisch einer Trennanlage (34) zugeleitet wird, ein Olefine und inerte Gaskomponenten enthaltender Zusatzstrom in eine der Zahl der Einsatzteilströme entsprechende Zahl von Zusatzteilströmen aufgeteilt und jeder Zusatzteilstrom in einen korrespondierenden Einsatzteilstrom bzw. in das aus einer Festbettzone austretende und der nächst folgenden Festbettzone aufgegebene Reaktionsgemisch eingeleitet wird.

## Claims

1. Process for the preparation of C₂- to C₄-olefins, preferably propylene, from a feed stream comprising vapour-form oxygenates, preferably methanol and/or dimethyl ether, and steam and having a temperature of from 280 to 480°C, which is passed through at least one fixed-bed zone (3) arranged in a reactor (2) and formed from a bed of granular, shape-selective zeolite catalyst of the pentasil type, and the oxygenates are catalytically converted into olefins with high selectivity for low olefins at a reaction temperature of from 350 to 550°C, preferably 420°C to 490°C, and the reaction mixture exiting the reactor is cooled in a cooler (6), so that water and hydrocarbons, in particular petrol, condense out, and said reaction mixture is separated in a separator (8) into a first product stream comprising C₂- to C₃-olefins, into a second product stream comprising C₄₊-olefins and into a third product stream comprising aqueous oxygenates, wherein propylene is separated from the first product stream by distillation and discharged from the process as main product, and wherein the third product stream comprising aqueous oxygenates is discharged from the process, **characterised in that** a reaction pressure of from 1.0 to 3.0 bara prevails at the inlet of the feed stream into the fixed-bed zone (3) and a reaction pressure of from 0.5 to 2.0 bara prevails at the outlet of the reaction mixture from the fixed-bed zone, and the temperature of the catalytic conversion is regulated in accordance with a target temperature value in the range from 440 to 520°C specified for the reaction mixture exiting the fixed-bed zone by means of a supplementary stream introduced into the feed stream and consisting of olefins and inert gas components, wherein the second product stream comprising C₄₊-olefins is separated by distillation into a stream comprising C₆-olefins and into a stream comprising C₇-olefins, wherein the C₇-olefins are discharged from the process and the C₆-olefins of the second product stream are combined with the inert gas components and the ethylene of the first product stream and recycled into the feed stream as supplementary stream, wherein the temperature of the feed stream at the inlet into the fixed-bed zone is determined and compared with the target temperature value for the reaction mixture exiting the fixed-bed zone and thus the temperature of the feed stream is influenced by the supplementary stream introduced into the feed stream with respect to the temperature of the catalytic conversion.

2. Process according to claim 1, **characterised in that** the target temperature value is in the range of 480 to 495°C.

3. Process according to any one of claims 1 to 2, **characterised in that** one or more of the substances steam, nitrogen, helium, neon, argon, hydrogen, carbon monoxide, carbon dioxide and C₁- to C₄-paraffins are used as inert gas components.

4. Process according to any one of claims 1 to 3, **characterised in that** the supplementary stream comprises one or more of the components C₂- to C₈-olefins, preferably C₂-olefins and C₄- to C₆-olefins, C₁- to C₈-paraffins, preferably C₁-to C₆-paraffins, C₅- to C₈-naphthenes, preferably C₅- to C₆-naphthenes, C₆- to C₇-aromatic compounds.

5. Process according to any one of claims 1 to 4, **characterised in that** the throughput of the oxygenates present in the feed stream is from 0.1 to 10 kg, preferably 0.3 to 1.5 kg per hour and per kg of catalyst employed.

6. Process according to any one of claims 1 to 5, **characterised in that** the feed stream fed to a reactor (23) having several, preferably two to six, fixed-bed zones (24, 25, 26) is divided into a number of feed sub-streams corresponding to the number of fixed-bed zones, and each feed sub-stream is fed to a corresponding fixed-bed zone, the reaction mixture delivered from a fixed-bed zone is fed to the next fixed-bed zone, and the reaction mixture delivered from the final fixed-bed zone in the downstream direction is fed to a separation unit (34), a supplementary stream comprising olefins and inert gas components is divided into a number of supplementary sub-streams corresponding to the number of feed sub-streams, and each supplementary sub-stream is fed into a corresponding feed sub-stream or into the reaction mixture exiting a fixed-bed zone and fed to the next fixed-bed zone.

## Revendications

1. Procédé de préparation d'oléfines en C₂ à C₄, de préférence de propylène, à partir d'un composé oxygéné sous forme de vapeur, de préférence du méthanol et/ou du diméthyléther et de la vapeur d'eau contenant un flux d'alimentation présentant une température allant de 280 à 480 °C, qui est dirigé à travers au moins une zone à lit fixe (3) formée à partir d'un amas granuleux émanant d'un catalyseur zéolithe granuleux de forme sélective de type pentasil disposée dans un réacteur (2) et les composés oxygénés ayant subi une réaction catalytique à une température de réaction allant de 350 à 550 °C, de préférence de 420 à 490 °C par rapport à des oléfines de plus haute sélectivité pour obtenir des oléfines plus faibles et le mélange de réaction émanant du réacteur est refroidi dans un dispositif de refroidissement (6), de sorte que l'eau et des hydrocarbures, en particulier du carburant, sont condensés, et est séparé dans un séparateur (8) dans un premier flux de produit contenant des oléfines en C₂ à C₃, dans un deuxième flux de produit contenant des oléfines en C₄₊ et dans un troisième flux de produit contenant un composé oxygéné aqueux, le propylène étant séparé par distillation d'un premier flux de produit et étant évacué du procédé en tant que produit principal et le troisième flux de produit contenant un composé oxygéné aqueux étant évacué du procédé, **caractérisé en ce que** prévalent à l'entrée du flux d'alimentation dans la zone de lit fixe (3) une pression réactionnelle de 1,0 à 3,0 bars et à la sortie du mélange réactionnel de la zone de lit fixe une pression réactionnelle de 0,5 à 2,0 bars et la température de la réaction catalytique suivant une valeur nominale de la température situé dans la plage allant de 440 à 520 °C prédéfinie pour le mélange réactionnel émanant de la zone de lit fixe est régulée par un flux auxiliaire se composant d'oléfines et de composants gazeux inertes introduit dans le flux d'alimentation, le deuxième flux de produit comprenant des oléfines en C₄₊ étant séparés par distillation dans un flux contenant des oléfines en C₆ et contenant des oléfines en C₇₊, les oléfines en C₇₊ étant évacuées du procédé et les oléfines en C₆ du deuxième flux de produit étant réunies avec les composants gazeux inertes et l'éthylène du premier flux de produit et étant remis en tant que flux auxiliaire dans le flux d'alimentation, la température du flux d'alimentation étant mesurée à l'entrée de la zone de lit fixe et comparée à la valeur nominale de la température pour le mélange réactionnel émanant de la zone de lit fixe et ainsi la température du flux d'alimentation par rapport à la température de la réaction catalytique est influencée par le flux auxiliaire introduit dans le flux d'alimentation.

2. Procédé selon la revendication 1, **caractérisé en ce que** la valeur nominale de la température se situe dans la plage allant de 480 à 495 °C.

3. Procédé selon l'une quelconque des revendications 1 à 2, **caractérisé en ce qu'**une ou plusieurs des substances suivantes : vapeur d'eau, azote, hélium, néon, argon, hydrogène, monoxyde de carbone, dioxyde de carbone et paraffine en C₁ à C₄ sont utilisées en tant que composants gazeux inertes.

4. Procédé selon l'une quelconque des revendications 1 à 3, **caractérisé en ce que** le flux auxiliaire contient un ou plusieurs des composants suivants : oléfines en C₂ à C₈, de préférence oléfines en C₂ et oléfines en C₄ à C₆, paraffines en C₁ à C₈, de préférence paraffines en C₁ à C₆, naphthènes en C₅ à C₈, de préférence naphthènes en C₅ à C₆ et composés aromatiques en C₆ à C₇.

5. Procédé selon l'une quelconque des revendications 1 à 4, **caractérisé en ce que** le débit du composé oxygéné contenu dans le flux d'alimentation va de 0,1 à 10 kg, de préférence de 0,3 à 1,5 kg par heure et par kg de catalyseur utilisé.

6. Procédé selon l'une quelconque des revendications 1 à 5, **caractérisé en ce que** le flux d'alimentation acheminé avec plusieurs, de préférence deux à six zones de lit fixe (24, 25, 26) dans un réacteur (23) est divisé en un nombre du nombre correspondant aux zones de lit fixe des flux partiels d'alimentation et est ajouté à chaque flux partiel d'alimentation d'une zone de lit fixe correspondante, le mélange réactionnel évacué d'une zone de lit fixe est fourni à la zone de lit fixe suivante et le mélange réactionnel évacué de la dernière zone de lit fixe situé en aval est acheminé dans un appareil de tamisage (34), un flux auxiliaire contenant une oléfine et des composants gazeux inertes en un nombre du nombre correspondant aux flux partiels d'alimentation étant séparé des flux partiels auxiliaires et chaque flux partiel auxiliaire dans un flux partiel d'alimentation correspondant ou est introduit dans le mélange réactionnel émanant d'une zone de lit fixe et de la zone de lit fixe suivante.
